# EUROPÄISCHE PATENTSCHRIFT

(11) **EP 1 765 388 B1**
(45) Veröffentlichungstag und Bekanntmachung des Hinweises auf die Patenterteilung: **17.04.2013**
(21) Anmeldenummer: 05767978.9
(22) Anmeldetag: 06.07.2005
(51) Int. Cl.: A61P 25/28, A61K 39/00, A61K 38/08, A61M 1/34

(54) **KOMBINATIONSTHERAPIE ZUR VORBEUGUNG ODER BEHANDLUNG DER ALZHEIMERSCHEN ERKRANKUNG SOWIE SET HIERFÜR**
COMBINATION THERAPY FOR PREVENTING OR TREATING ALZHEIMER'S DISEASE, AND KIT THEREFOR
THERAPIE COMBINEE POUR LA PREVENTION OU LE TRAITEMENT DE LA MALADIE D'ALZHEIMER, ET KIT CORRESPONDANT

(30) Priorität: 13.07.2004 AT 11852004
(43) Veröffentlichungstag der Anmeldung: 28.03.2007
(73) Patentinhaber: Affiris AG, 1030 Wien (AT)
(72) Erfinder: MATTNER, Franck, A-1190 Wien (AT); SCHMIDT, Walter, A-1020 Wien (AT)
(74) Vertreter: Alge, Daniel
(86) Internationale Anmeldenummer: PCT/EP2005/053224
(87) Internationale Veröffentlichungsnummer: WO 2006/005706

(56) Entgegenhaltungen:
- WO-A-00/72880
- WO-A-03/000719
- WO-A-03/051374
- WO-A-2004/013172
- WO-A-2004/056318
- WO-A-2004/062556
- WO-A-2005/025651
- WO-A-2006/005707
- US-B1- 6 551 266
- MCLAURIN J ET AL: "Therapeutically effective antibodies against amyloid-beta peptide target amyloid-beta residues 4-10 and inhibit cytotoxicity and fibrillogenesis" NATURE MEDICINE, NATURE PUBLISHING GROUP, NEW YORK, NY, US, Bd. 8, Nr. 11, November 2002 (2002-11), Seiten 1263-1269, XP002288573 ISSN: 1078-8956
- HEPPNER F L ET AL: "CURRENT CONCEPTS AND FUTURE PROSPECTS FOR ALZHEIMER DISEASE VACCINES" ALZHEIMER DISEASE AND ASSOCIATED DISORDERS, RAVEN PRESS, NEW YORK, NY, US, Bd. 18, Nr. 1, Januar 2004 (2004-01), Seiten 38-43, XP008057356 ISSN: 0893-0341
- REINEKE U ET AL: "Identification of distinct antibody epitopes and mimotopes from a peptide array of 5520 randomly generated sequences" JOURNAL OF IMMUNOLOGICAL METHODS, ELSEVIER, AMSTERDAM, NL, Bd. 267, Nr. 1, 1. September 2002 (2002-09-01), Seiten 37-51, XP004372978 ISSN: 0022-1759

## Beschreibung

Die Erfindung betrifft eine Kombinationstherapie zur Vorbeugung oder Behandlung der Alzheimer'schen Erkrankung und eink Set zur Durchführung dieser Kombinationstherapie.

Amyloid-β-Peptid (Aβ) spielt in der Neuropathologie der Alzheimer-Krankheit (AE) eine zentrale Rolle (Roher et al., 1993: "β-Amyloid-(1-42) is a major component of cerebrovascular amyloid deposits: Implications for the pathology of Alzheimer's disease [β-Amyloid-(1-42) ist eine Hauptkomponente von zerebrovaskulären Ablagerungen: Implikationen für die Pathologie der Alzheimer-Krankheit]" PNAS 90: 10836). Familiäre Formen der Erkrankung sind mit Mutationen im Amyloid-Vorläuferprotein (amyloid precursor protein, APP) und den Präsenilin-Genen in Verbindung gebracht worden. Mit der Erkrankung verbundene Mutationen in diesen Genen führen zu einer erhöhten Produktion der 42-Aminosäureform des Peptids (Aβ42), welches die Hauptform ist, die in den Amyloid-Plaques der Alzheimer-Krankheit zu finden ist. Ein Tiermodell der Erkrankung ist im Handel erhältlich. Die PDAPP transgene Maus, die mutantes humanes APP überexprimiert (wobei die Aminosäure in Position 717 F anstelle von V ist), entwickelt progressiv viele der neuropathologischen Kennzeichen der Alzheimer-Krankheit in Alters- und Gehirn-abhängiger Weise (Games et al., 1995: "Alzheimer-type neuropathology in transgenic mice overexpressing V717F β-amyloid precursor protein [Neuropathologie vom Alzheimer-Typ bei transgenen Mäusen, die V717F β-Amyloid-Vorläuferprotein überexprimieren]", Nature 373: 523).

Es sind bereits Vakzinationsstudien mit einem "normalen", nicht auf einem Mimotop basierenden Impfstoff durchgeführt worden. Transgene Tiere wurden mit aggregiertem Aβ42 immunisiert, entweder vor Einsetzen der AE-typischen Neuropathologien (6 Wochen) oder in höherem Alter (11 Monate): Die Immunisierung der jungen Tiere verhinderte die Entwicklung der Plaquebildung, neuritische Dystrophie und Astrogliose. Die Behandlung der älteren Tiere reduzierte die AE-artigen Neuropathologien deutlich. Dieser experimentelle Impfansatz löste die Entwicklung von Antikörpern gegen Aβ42 aus, die in der Lage waren, die Blut-Hirn-Schranke zu durchbrechen und Amyloid-Plaques anzugreifen (Schenk et al., 1999: "Immunization with amyloid-β attenuates Alzheimer-diseaselike pathology in the PDAPP mouse [Die Immunisierung mit Amyloid-β schwächt die Alzheimer-artige Pathologie bei der PDAPP-Maus ab]", Nature 400: 173). Die Plaques werden in der Folge durch mehrere Mechanismen entfernt, einschließlich durch Fc-Rezeptor mediierte Phagozytose (Bard et al., 2000: "Peripherally administered antibodies against amyloid β-peptide enter the central nervous system and reduce pathology in a mouse model of Alzheimer disease [Peripher verabreichte Antikörper gegen Amyloid-β-Peptid dringen in das Zentralnervensystem ein und reduzieren die Pathologie in einem Mäusemodell der Alzheimer-Krankheit]", Nature Med. 6: 916). Dieser Impfstoff war auch in der Lage, Erinnerungsdefizite aufzuschieben (Janus et al., 2000: "Aβ peptide immunization reduces behavioural impairment and plaques in a model of Alzheimer's disease [Aß-Peptid-Immunisierung reduziert Verhaltensbeeinträchtigungen und Plaques in einem Modell der Alzheimer-Krankheit]", Nature 408: 979).

Seit Ende 1999 ist eine äußerst vielversprechende Immunisierungstherapie für AE in der klinischen Testphase. Es wird angenommen, dass die Immunisierung das Immunsystem dazu bringt, die Plaques anzugreifen und diese Ablagerungen aus dem betroffenen menschlichen Gehirn zu entfernen, obwohl der zugrunde liegende genaue Mechanismus noch mehr im Detail charakterisiert werden muss.

Diese klinischen Versuche wurden von der pharmazeutischen Firma Elan zusammen mit ihrer Schwesterfirma American Home Products durchgeführt (therapeutischer Impfstoff AN-1792, QS21 als Adjuvans). Die Versuche der Phase I wurden im Jahre 2000 erfolgreich abgeschlossen. Gegen Ende 2001 wurde mit den Versuchen der Phase II begonnen, um die Wirksamkeit an einer Gruppe von Patienten mit schwacher bis mittlerer AE zu testen.

Nun wurden diese Versuche der Phase II wegen Nervenentzündungen bei mehreren Patienten ständig unterbrochen (Editorial 2002, "Insoluble problem? [Unlösbares Problem?]", Nature Med. 8: 191). Die Symptome umfassten aseptische Meningoenzephalitis, die zu einem sofortigen Stopp dieser weltweiten Versuche führte. Im schlechtesten Fall wird sich zeigen, dass die betroffenen Patienten eine Autoimmunantwort aufgebaut haben - ein Risiko, das viele Immuntherapien mit sich bringen. Autoimmunkomplikationen hätten angesichts der Allgegenwart von APP vorhergesehen werden können, das natürlich antigene Determinanten trägt, die es mit seinem proteolytischen Produkt gemeinsam hat. Seitdem haben sich zusätzliche Studien auf die Eigenschaften von aggregierten Aβ42 Immunisierungs-induzierten Antikörpern (bei Menschen und Mäusen) konzentriert und herausgefunden, dass die meisten Antikörper eine kleine Domäne zwischen Aminosäure 4 und 10 von Aβ42 (Aβ4-10) erkennen. Die Maus-Antikörper waren in der Lage, die Aß-Fibrillogenese zu blockieren, und zerstörten bereits vorhandene Aß-Fasern (McLaurin et al., 2002: "Therapeutically effective antibodies against amyloid-β peptide target amyloid-β residues 4-10 and inhibit cyctotoxicity and fibrillogenesis [Therapeutisch wirksame Antikörper gegen Amyloid-β-Peptid zielen auf Amyloid-β-Reste 4-10 und inhibieren die Zytotoxizität und die Fibrillogenese]", Nature Med. 8: 1263). Interessanter Weise reagieren die menschlichen Antikörper nicht mit dem auf der Oberfläche von Zellen exponierten APP oder irgendeinem anderen nicht aggregierten proteolytischen Produkt des Vorläufers (Hock et al., 2002: "Generation of antibodies specific for β-amyloid by vaccination of patients with Alzheimer disease [Die Erzeugung von β-Amyloid-spezifischen Antikörpern durch Impfung von Patienten mit Alzheimer-Krankheit]", Nature Med. 8: 1270). Es wurde ein deutlicher Unterschied zwischen den Seren von Menschen und Mäusen beobachtet: Im Gegensatz zu den menschlichen Antikörpern erkennen Mäuseantikörper monomere, oligomere und fibrilläre Aβ. Dies ist bedeutsam und kann eine Vorbedingung für die therapeutische Wirksamkeit sein, da es immer mehr Anzeichen dafür gibt, dass kleine Oligomere von Aβ, die von humanem anti-Aβ nicht erkannt werden, die toxischen Hauptakteure bei der Erkrankung sind (Walsh et al., 2002: "Naturally secreted oligomers of amyloid β protein potently inhibit hippocampal long-term potentiation in vivo [Natürlich sekretierte Oligomere von Amyloid β-Protein inhibieren sehr wirksam die Langzeit-Potenzierung im Hippocampus in vivo]", Nature 416: 535). Daher besteht eine potenzielle neue Strategie in der Immunisierung mit einem Impfstoff, der β-Amyloid-Aminosäuren 4-10 (anstelle von aggregiertem Aβ42) enthält. Trotz der noch unbekannten Wirksamkeit kann sich auch diese Strategie mit Autoimmunproblemen konfrontiert sehen, da die Patienten direkt mit einem (linearen B-Zellen-) "Selbst"-Epitop immunisiert werden sollen.

Trotz dieser enttäuschenden Entwicklungen bei den jüngsten AE-Impfstrategien wird ein Aβ-Impfstoff noch immer als der verheißungsvollste Weg im Kampf gegen AE angesehen. Es sind jedoch dringend Verbesserungen und neue Strategien bei der AE-Impfung erforderlich. Insbesondere sollte ein solcher Impfstoff keine autoreaktiven T- und/oder B-Zellen anregen.

Es werden aber auch zunehmend andere Therapeutika entwickelt, die die Amyloid-ß-Produktion, die Amyloid-ß-Aggregation oder die von diesen Aggregaten verursachten neurotoxischen Vorkommnisse verhindern sollen. Eine zusammenfassende Darstellung der bislang eingeschlagenen therapeutischen Strategien für AE ist im Übersichtsartikel von Wolfe (Nature Reviews Drug Discovery 1 (2002) 859-866) gegeben.

Die Amyloid-ß-Plaques bilden sich ausgehend vom so genannten Amyloid-ß Precurser Protein (APP), welches ein integrales Transmembran-Protein ist (für das auch keine bekannte physiologische Funktion klar belegt ist; allerdings lassen neueste Forschungsergebnisse vermuten, dass APP als sog. Membran-Cargo-Rezeptor für Kinesin I fungiert). APP wird durch so genannte Sekretasen proteolytisch gespalten, wobei physiologisch vor allem ein 40-Aminosäuren langes Aß-Peptid (Aß40) gebildet wird. Andere, kürzere und längere Formen von Aß entstehen ebenfalls, vor allem eine 42-Aminosäure-Version (Aß42), welche ein hohes Aggregationsvermögen aufweist. Diese Aß42-Form ist daher auch die überwiegende Form in amyloiden Plaques. Die für diese unterschiedlichen Spaltungen verantwortlichen Sekretasen (α-, und vor allem ß- und gamma-Sekretase) bilden daher auch primäre Angriffsziele einer möglichen AE Behandlungsstrategie. Es wurde daher versucht, Modulatoren bzw. Inhibitoren für diese Enzyme bei der Behandlung von AE einzusetzen (wie z.B. Benzodiazepine, Sulphonamide, Benzocaprolactame).

Ein weiteres Gen, das mit AE assoziiert ist, ist Apolipoprotein E, wobei hierfür drei allele Varianten existieren (APOE2, APOE3 und APOE4). Es hat sich gezeigt, dass Personen mit ein oder zwei Kopien von APOE4 ein höheres Risiko für AE aufweisen, wohingegen APOE2-Träger ein geringeres Risiko, verglichen mit der Gesamtbevölkerung, aufweisen. Auch zeigt es sich, dass Personen, die Statine, also Arzneimittel, die Cholesterinbiosynthese inhibieren, zu sich nehmen, ein deutlich verringertes Risiko für AE aufweisen. Eine weitere Strategie zur Behandlung von AE konzentriert sich daher auf die Inhibition der Cholesterinbiosynthese, eben mit beispielsweise Statinen.

Ein weiterer Ansatz zur Behandlung von AE betrifft die Inhibition der Amyloid-Aggregation in zerebralen Plaques, welche unter anderem ebenfalls durch Sekretase-Inhibitoren durchgeführt werden könnte. Weiters wurde auch vorgeschlagen, den Zinkgehalt zu senken, da Zink in physiologisch relevanten Konzentrationen die Aggregation von Aß induzieren kann.

Weitere AE-Behandlungsstrategien, die im Stand der Technik vorgeschlagen worden sind, betreffen die Verhinderung der APP-Expression und die Erhöhung der Aß-Clearance, wobei für erstere Substanzen gesucht wurden, die mit der APP-Promoter-Region wechselwirken. Im Hinblick auf die Aß-Clearance wurde eine Erhöhung der Aktivität von bestimmten Proteasen, wie das Insulin abbauende Enzym und Neprolysin, oder die periphere Applikation von anti-Aβ-Antikörpern (De Mattos et al., PNAS 98 (15)(2001), 8850-8855) vorgeschlagen. Derartige Versuche brachten allerdings bereits im Maus-Modell widersprüchliche Ergebnisse (Wolfe (2002)). Schließlich wurde auch versucht, bereits bestehende Amyloid-Plaques wieder aufzulösen, beispielsweise durch Erniedrigung des Amyloid ß-Niveaus im Serum von AE-Patienten. In diesem Zusammenhang wurde auch vorgeschlagen, die Plaque-Ablagerungen von ß-Amyloid-Proteinen im Gehirn durch Apherese-Verfahren zu reduzieren (US 6 551 266, worin die Entfernung von Makromolekülen mit einem Molekulargewicht von mehr als 500kD durch Apherese vorgeschlagen wird), ohne dass dies allerdings für AE auch tatsächlich gezeigt wurde. Die Auflösung durch bereits bestehende Plaques in Hirnzellen ist aber durch Aphereseverfahren direkt nicht möglich (Blut/Hirn-Schranke ist unüberwindlich für Plaques oder auch Moleküle mit >500kD).

Wie erwähnt, ist die Anwesenheit von ß-Amyloid(Aß40 und Aß42)-Plaques das am meisten ins Auge fallende pathologische Merkmal der AE. Reduktion von Aß wird daher als das primäre pharmazeutische Ziel bei der AE Prophylaxe und -Therapie angesehen. Trotz der beschriebenen Amyloid-Entfernung durch Induktion von anti-Aß-Antikörpern durch aktive Immunisierung blieben klinische Versuche bei dieser Immunisierung aufgrund von schweren Nebenwirkungen, die zum Abbruch der Behandlung führten, bislang erfolglos. Jüngere präklinische Ergebnisse zeigten, dass die Antikörper (auch) zur peripheren Reduktion von Aß führen können und so möglicherweise die Aß-Peripherie-Gehirn-Dynamik verändern können.

Es wurde weiters gezeigt, dass eine periphere Behandlung mit einem Mittel, das eine hohe Affinität zu Aß hat (wie z.B. Gelsolin oder GM1), die Menge von Aß im Gehirn erniedrigt (Masouka et al, Journal of Neuroscience 2003: 29-33). Demgemäß wurde als allgemeiner Ansatz vorgeschlagen, Verbindungen, die den Aß-Gehalt im Plasma verringern können, Amyloidose im Gehirn verringern oder verhindern können. Dadurch könnten neue therapeutische Mittel entwickelt werden, deren Wirkung nicht auf die Überwindung der Blut/Hirn-Schranke angewiesen ist.

Für diesen Plasma-Aß-Sequestrations-induzierten Aß-Efflux aus dem Hirn wurde ein Verfahrens-abhängiger Effekt auf den Plasma Aß-Gehalt gezeigt: Der Aß-Gehalt in Plasma wurde durch Gelsolin nicht gesenkt; Verabreichung von Gelsolin und passive Immunisierung mit anti-Aß-monoklonalen Antikörpern führte stattdessen zu erhöhtem Aß-Gehalt in Plasma. Die Aß-Last im Gehirn wurde allerdings nur bei Verwendung relativ junger APPtransgener Mäuse im Experiment reduziert; bei Verwendung von Mäusen, die älter als 6 Monate waren, stellte sich die Behandlung als wirkungslos heraus. Dies könnte auf die erhöhte Unlöslichkeit von Aß im Gehirn älterer Mäuse zurückzuführen sein. Andererseite könnte eine längere Behandlungsdauer möglicher Weise doch erfolgreich sein, jedoch sind weder die Verabreichung von Gelsolin oder GM1 noch die passive Immunisierung für eine Langzeit-Verabreichung geeignet.

Die WO 2004/056318 beschreibt Therapien zur Behandlung der Alzheimer'schen Erkrankung, darunter die Verabreichung von Aß-bindenden Substanzen sowie Dialyse, inklusive Hämodialyse und Plasmaperfusion.

Aufgabe der vorliegenden Erfindung war daher die Bereitstellung einer neuen Behandlungs- und Präventionsstrategie für die Alzheimer'sche Erkrankung, insbesondere einer Strategie, die auch auf einer erfolgreichen Immunisierung beruht.

Demgemäß wird mit der vorliegenden Erfindung ein Set wie in Anspruch 1 definiert, zur Verfügung gestellt. Dabei wird erfindungsgemäß der Aß-Efflux induziert (durch Mittel, wie z.B. Gelsolin, GM1, ein Aß-spezifisches aktives oder passives Vakzin) und dieser Efflux durch eine Aß-Apherese aufrecht erhalten. In diesem Zusammenhang ist sogar eine ein-oder zweimalige aktive Immunisierung mit einer Vakzine, enthaltend Aß, Aß-Derivate oder Aß-Mimotope, ausreichend, um eine IgM und/oder IgGmediierte Sequestration von Plasma Aß zu induzieren.

Vorzugsweise kommt im Set als Apherese-Träger eine sterile und pyrogenfreie Säule zum Einsatz.

Im Set ist das Mittel zur Induzierung einer Sequestration von Amyloid ß (Aß) in Plasma vorzugsweise ausgewählt aus Mitteln mit einer hohen Affinität zu Aß, d.h Gelsolin oder GM1, einer Aß-spezifischen aktiven oder passiven Vakzine oder Aß-spezifischen humanisierten monoklonalen Antikörpern.

Das Aß-spezifische aktive Vakzin ist dabei vorzugsweise ein Peptid gemäβ Anspruch 3, auch Mimotop gennant.

Besonders geeignete Epitope sind ausgewählt aus mindestens einem der folgenden Epitopen: EIDYHR, ELDYHR, EVDYHR, DIDYHR, DLDYHR, DVDYHR, DIDYRR, DLDYRR, DVDYRR, DKELRI, DWELRI, YREFFI, YREFRI, YAEFRG, EAEFRG, DYEFRG, ELEFRG, DRELRI, DKELKI, DRELKI, GREFRN, EYEFRG, DWEFRDA, SWEFRT, DKELR, SFEFRG, DAEFRWP, DNEFRSP, GSEFRDY, GAEFRFT, SAEFRTQ, SAEFRAT, SWEFRNP, SWEFRLY, SWELRQA, SVEFRYH, SYEFRHH, SQEFRTP, SSEFRVS, DWEFRD, DAELRY, DWELRQ, SLEFRF, GPEFRW, GKEFRT, AYEFRH, DKE (Nle) R, DKE(Nva)R oder DKE (Cha)R.

Erfindungsgemäß wird ein Aβ42 Mimotop zur Impfung gegen AD verwendet: Das Mimotop löst die Produktion von Antikörpern gegen Aβ42, jedoch nicht gegen das native APP aus. Das Mimotop kann mit einem (monoklonalen) Antikörper und (im Handel erhältlichen) Peptidbibliotheken identifiziert werden (z.B. gemäß Reineke et al., 2002: "Identification of distinct antibody epitopes and mimotopes from a peptide array of 5520 randomly generated sequences [Identifizierung distinkter Antikörperepitope und Mimotope von einer Peptidsammlung von 5520 willkürlich hergestellten Sequenzen]", J. Immunol. Methods 267: 37). Es wird ein (monoklonaler) Antikörper verwendet, der APP nicht erkennt, jedoch nur verschiedene Aß-Spezies mit Amino-terminaler Asparaginsäure erkennt (ein Beispiel für einen solchen Antikörper ist in Johnson-Wood et al., 1997, beschrieben: "Amyloid precursor protein processing and Aβ42 deposition in a transgenic mouse model of Alzheimer disease [Die Verarbeitung von Amyloid-Vorläuferprotein und die Ablagerung von Aβ42 in einem transgenen Maus-Modell der Alzheimer-Krankheit]", PNAS 94: 1550). Ein solcher Antikörper hat sich im Laufe der vorliegenden Erfindung als ein ideales Werkzeug zur Identifizierung von für die Impfung geeigneten Mimotopen erwiesen. Obwohl sich gezeigt hat, dass derartige monoklonale Antikörper in einem Maus-Modell von AD positive Wirkungen haben, wenn sie direkt an die Mäuse verabreicht werden (Bard et al., 2000; "Peripherally administered antibodies against amyloid β-peptide enter the central nervous system and reduce pathology in a mouse model of Alzheimer disease [Peripher verabreichte Antikörper gegen Amyloid β-Peptid dringen in das Zentralnervensystem ein und verringern die Pathologie in einem Maus-Modell der Alzheimer-Krankheit]", Nature Med. 6: 916), ist niemals vorgeschlagen worden, diese Antikörper als Mimotop-Suchwerkzeuge zur Isolierung von AD-Impfstoffverbindungen zu verwenden.

Im Stand der Technik konzentrierten sich alle Bemühungen auf die natürlich vorkommenden Aß-Peptide. Wie oben erwähnt, wurden die klinischen Versuche mit Aß-Peptid-Impfstoffen auf Grund von Nervenentzündungsereignissen gestoppt. Tatsächlich schlagen T-Zellen-Epitop-Vorhersageprogramme (BIMAS für MHC-Klasse I-beschränkte Epitope und TEPITOPE für MHC-Klasse II-beschränkte Epitope) eine große Anzahl von (Selbst-)Epitopen innerhalb der Sequenz vor. Dies könnte implizieren, dass die Nervenentzündungsereignisse durch Autoimmunreaktionen ausgelöst wurden, was einen solchen Impfstoff für die allgemeine Anwendung ungeeignet machen würde.

Im Gegensatz zu solchen Aß-Impfstoffen, die im Stand der Technik vorgeschlagen wurden, ist während der Behandlung mit einem Impfstoff, der ein erfindungsgemäßes Mimotop enthält, kein Auftreten von Autoimmunreaktionen zu erwarten, da der erfindungsgemäß für die Mimotopenidentifizierung verwendete (monoklonale) Antikörper APP nicht erkennt und sich die Mimotopsequenz von von Aβ42 abgeleiteten Selbstsequenzen unterscheidet, die in Versuchen bisher verwendet wurden oder in zukünftigen Versuchen verwendet werden sollen.

Der erfindungsgemäß für die Mimotopenidentifizierung verwendete Antikörper erkennt die von Aβ abgeleitete Aminosäuresequenz DAEFRH (= ursprüngliches Epitop) mit einer freien Amino-terminalen Asparaginsäure, natives APP jedoch nicht. Der Antikörper kann ein monoklonales oder polyklonales Antikörperpräparat oder jeglicher Antikörperteil oder Derivat davon sein, die einzige Vorbedingung ist, dass das Antikörpermolekül spezifisch das DAEFRH-Epitop erkennt, d.h. dass es nicht an die natürlichen N-terminal verlängerten Formen des Amyloid-Vorläuferproteins bindet, was bedeutet, dass die Bindungsfähigkeit an das DAEFRH-Epitop mindestens 100 Mal, vorzugsweise mindestens 1000 Mal, noch mehr bevorzugt mindestens 105 Mal höher ist als an das APP-Molekül. Der Antikörper kann ein Antikörper sein, der die gleiche oder eine höhere Bindungsfähigkeit an die DAEFRH-Sequenz aufweist als der Antikörper, der von Johnson-Wood et al. 1997 beschrieben wurde. Selbstverständlich können auch Antikörper mit einer geringeren Bindungsfähigkeit verwendet werden (> 10%, > 50% oder > 80% der Bindungsfähigkeit des Antikörpers von Johnson-Wood et al.), obwohl die höhere Bindungsfähigkeit mehr bevorzugt ist.

Die erfindungsgemäßen Verbindungen binden an jene Antikörper mit vergleichbarer Spezifität wie die DAEFRH-Sequenz.

Das erfindungsgemäß zu verwendende Mimotop kann im Impfstoff in isolierter (Peptid-)Form vorliegen oder kann an andere Moleküle gekoppelt oder komplexiert sein, wie pharmazeutische Trägersubstanzen oder Polypeptid-, Lipid- oder Kohlenhydratstrukturen. Die Mimotope können jedoch (kovalent oder nicht kovalent) an unspezifische Linker oder Träger gekoppelt sein, insbesondere Peptidlinker oder Proteinträger. Weiters können die Peptidlinker oder Proteinträger aus T-Zellen-Helfer-Epitopen bestehen oder solche enthalten.

Vorzugsweise ist der pharmazeutisch annehmbare Träger KLH, Tetanustoxoid, Albumin bindendes Protein, bovines Serumalbumin, ein Dendrimer (MAP; Biol. Chem. 358: 581) sowie die Adjuvanssubstanzen, die in Singh et al., Nat. Biotech. 17 (1999), 1075-1081 (insbesondere jene in Tabelle 1 dieses Dokuments), und O'Hagan et al., Nature Reviews, Drug Discovery 2 (9) (2003), 727-735 (insbesondere die darin beschriebenen endogenen immunpotenzierenden Verbindungen und die Abgabesysteme) beschrieben sind, oder Mischungen davon. Außerdem kann die Impfstoffzusammensetzung Aluminiumhydroxid enthalten.

Ein Impfstoff, der die vorliegende Verbindung (Mimotop) und den pharmazeutisch annehmbaren Träger umfasst, kann auf jegliche geeignete Anwendungsweise, z.B. i.v., i.p., i.m., intranasal, oral, subkutan, etc. und in jeglichen geeigneten Abgabevorrichtung (O'Hagan et al., Nature Reviews, Drug Discovery 2 (9), (2003), 727-735) verabreicht werden. Typischer Weise enthält der Impfstoff die erfindungsgemäße Verbindung in einer Menge von 0,1 ng bis 10 mg, vorzugsweise 10 ng bis 1 mg, insbesondere 100 ng bis 100 µg oder, alternativ dazu, z.B. 100 fMol bis 10 µMol, vorzugsweise 10 pMol bis 1 µMol, insbesondere 100 pMol bis 100 nMol. Der Impfstoff kann auch typische Hilfsstoffe, z.B. Puffer, Stabilisatoren, etc. enthalten.

Gemäß der vorliegenden Erfindung wird - zur Aufrechterhaltung des Aß-Efflux nach der Initiierung im Rahmen der Kombinationstherapie - eine Apherese-Vorrichtung, umfassend einen mit dem Blut oder Plasmafluss kontaktierbaren festen Träger, der einen Amyloid-ß-Precurser-Protein (APP) bindenden Rezeptor aufweist, zur Verfügung gestellt. Mit der vorliegenden Apherese-Vorrichtung kann gezielt bei AE-Patienten bzw. Personen mit AE-Risiko mittels Apherese eine Clearance von APP oder APP-Abbauprodukten, insbesondere Aß40 oder Aß42, vorgenommen und so der Effekt der Aß-Sequestrierung im ersten Schritt aufrecht erhalten werden. Es ist bekannt, dass ein dynamisches Äquilibrium von Aß42 zwischen dem zentralen Nervensystem (ZNS) und dem Plasma besteht. Es konnte - wie erwähnt - im Mausmodell gezeigt werden (DeMattos PNAS 2001, siehe oben), dass die periphere Applikation von anti-Aß-Antikörpern die ZNS und Plasma Aß42 Clearance beeinflusst und die Aß42 Belastung im Gehirn reduziert, ohne dass die anti-Aß-Antikörper die Blut-Hirn-Schranke überwinden. Diese Ergebnisse wurden von Matsuoka et al (Journal of Neuroscience 2003: 29-33) durch die periphere Applikation anderer Aß42 bindender Moleküle (Gelsolin und GM1) bestätigt. Damit kann der Prozess der Entstehung der Plaques im Gehirn an einer sehr gut zugänglichen Stelle unterbunden werden, nämlich bereits im Blut, d.h. diese Proteine bzw. Abbaupeptide können dann nicht mehr in das Gehirn zurück gelangen und dort aggregieren. Der Prozess der Entstehung der Plaques im Gehirn kann also durch Abfangen von Aß42 im Blut unterbunden werden. Hierbei ist es nicht kritisch, ob die Rezeptoren in der Apherese-Vorrichtung, die mit dem Blut oder Plasma des Patienten kontaktiert werden, spezifisch für Aß42 oder andere Abbauformen von APP sind, wesentlich ist nur, dass mit dieser spezifischen Adsorption APP und dessen (proteolytische) Abbauprodukte, insbesondere Aß42, aus dem Blut eliminiert werden, so dass es nicht zu einem "falschen" Proteinabbau (nämlich zu Aß42) oder zu einer Entstehung von Plaques kommt. Damit beruht die vorliegende Erfindung auf einem völlig anderen Anwendungsansatz für die Apherese als die US 6 551 266, nämlich auf der Eliminierung der potenziellen Plaque-Bausteine und nicht erst der Plaques. Im Übrigen scheidet die Eliminierung von Plaques mittels Apherese von vornherein als nicht effektiv zur AE-Behandlung aus, da die Blut-Apherese die Regionen der Plaqueentstehung im Gehirn gar nicht erreichen kann.

Auf der anderen Seite hat die erfindungsgemäße Kombinationstherapie gegenüber Verfahren, die im Körper selbst zur Abreicherung von Aß führen (wie z.B. in DeMattos et al., PNAS 98(15)(2001), 8850-8855 mit peripheren anti-Aß Antikörpern) und über längere Zeit durchgeführt werden, den entscheidenden Vorteil, dass hierbei keine Autoimmunantworten ausgelöst werden können. Weiters müssen erfindungsgemäß auch keine Substanzen dem Patienten zugeführt werden, die erst im Körper selbst wirken können (eventuell erst, nachdem sie an eine bestimmte Stelle transportiert worden), sondern das pathogene Agens wird gezielt entfernt, also die Ursache der Erkrankung spezifisch extrakorporal abgetrennt, ohne dass die Reaktionsprodukte im Körper eliminiert werden müssen.

Dabei können erfindungsgemäß die bereits bestehenden und bekannten Apherese-Vorrichtungen in allen Ausführungsformen leicht an die vorliegende Erfindung angepasst werden. Insbesondere sollte bei der Wahl des festen Trägers (und der Apheresevorrichtung) auf dessen (deren) medizintechnische Eignung Bedacht genommen werden. Derartige Träger, Verfahren oder Vorrichtungen sind unter anderem in der US 5 476 715, 6 036 614, 5 817 528 oder 6 551 266 beschrieben. Entsprechende kommerzielle Apherese-Apparate werden auch unter anderem von den Firmen Fresenius, Plasmaselect, ASAHI, Kaneka, Braun, etc. vertrieben, wie z.B. das LDL-Therasorb®, das Immunosorba®, das Prosorba®, das Globafin®, das Ig-Therasorb®, das Immusorba®, das Liposorba®, das HELP®, das DALI®, das Bilirubin-Gallensäure-Absorber BR-350, die Prometheus® Entgiftung, das MARS®, das ADAsorb-System von Medicap oder das Plasma FLO-System. All diese Systeme - obgleich in der kommerziellen Form nicht primär immer auf die spezifische Eliminierung eines einzelnen Proteins gerichtet - können ohne weiteres von einem Apherese-Fachmann an die vorliegende Erfindung angepasst werden, z.B. als Immunapherese und/oder unter Einbau des erfindungsgemäßen festen Trägers (z.B. als Säule) in das Apherese-Gerät.

Die erfindungsgemäßen "APP bindenden Rezeptoren" sind Substanzen, die eine Affinität zum Liganden APP und dessen biologischen Nebenprodukten, insbesondere Aβ42, haben und in der Lage sind, diese Polypeptide aus dem Blut oder Plasma von AE-Patienten oder Personen mit einem Risiko für AE zu entfernen.

Beispiele für anti-APP-Antikörper sind 3D6 (Aß1-5), 2H3 (Aß1-12), 2G3 (Aß33-40), 21F12 (Aß33-42), 12H7 (Aß33-42) (Johnson-Wood et al, PNAS 1997:1550-1555), 10D5, 16C11 (Bard et al, Nature Medicine 2000:916-919) die De Mattos et al. (2001) beschriebenen Antikörper (m266, m243) sowie Antikörper gleicher Spezifität. Derartige Antikörper werden beispielsweise bei der Immunisierung von Säugetieren mit Vakzinformulierungen, enthaltend APP, Aß42 oder Fragmente oder Varianten davon, erhalten, gegebenenfalls gefolgt von Zellfusion und Klonselektionsprotokollen (bei monoklonalen Antikörpern).

Gelsolin (Matsuoka et al 2003, siehe oben), apoJ und apoE (DeMattos et al 2001, siehe oben) sind weitere Beispiele für APP-bindende Protein-Rezeptoren. GM1 ist ein Beispiel für einen APP-bindenden Gangliosid-Rezeptor (Matsuoka et al 2003, siehe oben).

Weiters können auch APP-bindende Rezeptoren auf Basis von Nukleinsäuren ("Aptamere"; aber auch "Decoy"-Oligodeoxynuleotide (ds Oligonukleotide, die von der Sequenz her Bindungsstellen für Transkriptionsfaktoren darstellen)) eingesetzt werden, wobei auch diese mit verschiedensten (Oligonukleotid-) Bibliotheken (z.B. mit 2-180 Nukleinsäureresten) aufgefunden werden können (z.B. Burgstaller et al., Curr. Opin. Drug Discov. Dev. 5 (5) (2002), 690-700; Famulok et al., Acc. Chem. Res. 33 (2000), 591-599; Mayer et al., PNAS 98 (2001), 4961-4965, uvm.). Das Nukleinsäurerückgrat kann dabei beispielsweise durch die natürlichen Phosphordiester-Verbindungen aber auch durch Phosphorotioate oder Kombinationen oder chemische Variationen (z.B. als PNA) aufgefunden werden, wobei als Basen erfindungsgemäß vor allem U, T, A, C, G, H und mC eingesetzt werden können. Die 2'-Reste der Nukleotide, die gemäß der vorliegenden Erfindung eingesetzt werden können, sind vorzugsweise H, OH oder oder andere Schutzgruppen und Modifikationen an der 2'-Position, wobei die Nukleinsäuren auch modifiziert werden können, also beispielsweise mit Schutzgruppen, wie sie üblicherweise in der Oligonukleotidsynthese angewendet werden, versehen werden. Unter "Schutzgruppe" wird dabei eine Veretherung des Sauerstoffatoms verstanden, wohingegen bei der 2'-Modifikation die -OH-Gruppe durch etwas anderes ersetzt wird. Für beide Varianten bestehen im Stand der Technik sehr vielfältige Möglichkeiten, besonders bevorzugte Schutzgruppen sind dabei Methyl-, Allyl-, Propyl-, u. dgl. (also z.B. 2'-OCH3, 2'-O-CH=CH2, etc.); besonders bevorzugte Modifikationen sind 2'-Desoxy, 2'-Amino, 2'-Fluoro, 2'-Bromo, 2'-Azido, aber auch Metalle, wie Selen, etc.. Weiters können erfindungsgemäß auch die Oligonukleotid-Stabilisierungstechniken, die für die Antisense-Technologie (Ribozyme, RNAi, etc.) entwickelt wurden, zur Bereitstellung der Nukleinsäuren herangezogen werden (s.z.B. die dabei führenden Firmen ISIS und Ribozyme Pharmaceuticals, insbesondere deren Patentdokumente und Homepage).

APP bindende Aptamere (die erfindungsgemäß wie oben definiert auch Aß42 bindende Aptamere miteinschließen) sind daher ebenfalls bevorzugte APP bindende Rezeptoren im Rahmen der vorliegenden Erfindung.

Erfindungsgemäß werden daher die APP-bindenden Rezeptoren an ein geeignetes Trägermaterial zur extra-korporalen Eliminierung von APP und dessen proteolytischen Abbauprodukten in Alzheimer-(Risiko-)Patienten verwendet.

Bei der Anwendung der vorliegenden Erfindung in medizinischer Routinepraxis ist es erforderlich, dass der Träger steril und pyrogenfrei ist, so dass jede Trägersubstanz bzw. jede Rezeptor-/Träger-Kombination, die diese Merkmale erfüllt, erfindungsgemäß bevorzugt ist (siehe z.B. US 6 030 614 oder US 5 476 715). Zu den geeigneten Beispielen zählen poröse Homopolymere, Co- oder Terpolymere von Vinyl enthaltenden Monomeren (z.B. Acryl-Säure, wie z.B. TSK Toyopearl, Fractogel TSK), Träger mit Modifkationen (Aktivierungen) mit Oxiran enthaltenden Verbindungen (z.B. Epichlorohydrin) und gegebenenfalls weiteren Reaktionen mit NH3, Amino oder Carboxyl enthaltenden Verbindungen, oder CNBr oder CNCl-Adsorbientien, wie in der EP 110 409 A und der DE 36 17 672 A beschrieben. Besonders bevorzugte Adsorptionsmaterialien für therapeutische Zwecke sind geeignet, einen Verlust von Blutzellen zu vermeiden, aktivieren das Komplementsystem nicht oder nur geringfügig und halten eine Aggregatbildung im extra-korporalen Kreislauf möglichst hintan. Weiters sollten die eingesetzten Trägermaterialien vorzugsweise auch in rezeptorgekoppelter Form ausreichend stabil gegenüber Sterilisierungsmaßnahmen sein, insbesondere gegenüber Ethylen-Oxid-Sättigung, Glutaraldehyd-Sättigung, Gamma-Bestrahlung, Dampfbehandlung, UV-Behandlung, Lösungsmittelbehandlung und/oder Detergensbehandlung, etc.. Beispielsweise können auch Produkte auf Sepharose-, Agarose-, Acryl-, Vinyl-, Dextran- etc. -Basis eingesetzt werden, die vorzugsweise geeignete funktionelle Gruppen zur Anbindung der APP bindenden Rezeptoren bereits kommerziell erhältlich aufweisen. Weitere geeignete Träger schließen auch Monolithe ein (Träger auf Basis von quervernetzten Glycidylmethacrylat-coethylenglykoldimethacrylat-Polymer).

Zur Kopplung der Rezeptoren an die geeigneten Träger ist die dem Fachmann bekannte Chemie einsetzbar (z.B. Bioconjugate Techniques, Greg T Hermanson, Ed., Academic Press, Inc, San Diego, CA, 1995, 785pp).

Gemäß einem weiteren Aspekt betrifft die vorliegende Erfindung die Verwendung des erfindungsgemäßen Sets zur Bereitstellung einer Behandlung oder Behandlungsvorrichtung der Alzheimer'schen Erkrankung oder zur Vorbeugung einer derartigen Erkrankung im Rahmen der erfindungsgemäßen Kombinationstherapie, indem die Vorrichtung geeignet zur Behandlung des jeweiligen Patienten hergerichtet wird. Bei der Durchführung der Behandlung wird ein Patient für eine zur effektiven Eliminierung von APP-Polypeptiden ausreichende Zeitdauer an die Apherese-Apparatur angeschlossen, wobei der Blut- oder Plasmafluss des Patienten mit dem festen Träger, umfassend den APP bindenden Rezeptor, kontaktiert wird, worauf APP und/oder die proteolytischen Abbauprodukte von APP, insbesondere Aβ42, gebunden werden. Im Zuge der Apherese-Behandlung sind natürlich peripherer oder zentralvenöser Venenzugang bzw. arterievenöse Fistel sicherzustellen, ebenso wie ausreichende Antikoagulation, sowie die erforderlichen Quantifizierungs- und Messdaten aufzuzeichnen. Weiters wird bei den meisten Apherese-Verfahren eine Primär-Trennung von Plasma und Blutzellen vor der eigentlichen Plasmabehandlung erforderlich sein. Besondere Personen, bei denen eine vorbeugende Maßnahme erforderlich ist, sind familiär belastete Personen, ältere Personen (>50, >60 oder >70 Jahre) oder Personen mit einem anderen Risikofaktor für AE, insbesondere genetische Faktoren.

Die Erfindung wird anhand der nachfolgenden Beispiele, auf die sie selbstverständlich nicht eingeschränkt ist, näher erläutert.

### 1. Herstellung des APP-Rezeptor tragenden Trägers

### 1.1 Monolithische Säule

Eine CIM® Epoxy Monolithic Säule (BIA Separations, SI) wird gemäß den Angaben des Herstellers mit 0,5 M Na-Phosphat-Puffer bei pH 8,0 äquilibriert und ein monoklonarer Antikörper gegen Aβ Peptid ebenfalls gemäß Herstellerangaben aktiviert und an die CIM-Säule gekoppelt. Die Säule wird mehrfach mit Phosphat-Puffer gewaschen (+ 1 M NaCl) und überschüssige Epoxy-Gruppen gegebenenfalls noch blockiert.

Qualitätssicherung wird mittels Kontrolle im Wasch- und Äquilibrationseluat durchgeführt; nur Säulen ohne aktive Epoxy-Gruppen und ohne Antikörper-Leakage im Eluat werden weiterverwendet und in eine Apharese-Apparatur eingebaut.

### 1.2 Sepharose-Säule

Ein Agarose Bulk-Material (Sepharose CL4B) wird aseptisch in einen sterilen und pyrogenfreien Behälter gefüllt und das Material aseptisch gewaschen, wobei zwischen jedem Waschschritt das Gelmaterial völlig unter Vakuum getrocknet wird. Die Sepharose wird anschließend für 30 Minuten bei 115°C im Autoklaven dampfsterilisiert.

Nach der Sterilisierung wird die Sepharose in einem sterilen Behälter in 60% Aceton/Wasser aufgenommen und mit CNBr und Triethylamin aktiviert (14 g CNBr pro 96 ml Acton; 30 ml Triethylamin in 66,2 ml 87 %igem Aceton). Dann wurde eine Aceton/HCl-Lösung zugesetzt (392 ml steriles, pyrogenfreies Wasser; 16,3 ml 5 N HCl, 408 ml Aceton). Die aktivierte Sepharose wird gewaschen und innerhalb von 2 h der Kopplungsreaktion zugeführt, um die Hydrolyse von aktivierten Gruppen zu verhindern. Eine sterilfiltrierte Antikörper-Lösung (m266 bzw. m243) wird in das Reaktionsgefäß eingebracht und für mindestens 90 min gerührt. Schließlich wird die Reaktionslösung gründlich gewaschen (mit isotonischem Phosphat-Puffer), bis keine Reaktionsprodukte im Eluat nachweisbar sind, und die Antikörper-gekoppelte Sepharose in sterile und depyrogenisierte Glassäulen mit Glassintern gefüllt und einer abschließenden Qualitätskontrolle unterzogen (Eluatanalyse hinsichtlich Reaktionsprodukten, Schwermetallen etc.; Partikelanalyse, Pyrogenizität; Sterilität).

### 2. Tiermodell für die Apherese-Behandlung von Alzheimer-Patienten

In den letzten Jahren ist am "Gerhardt Katsch"-Diabetes-Institut in Karlsburg, DE, ein spezielles extrakorporales System für experimentelle Apherese in frei beweglichen kleinen Tieren entwickelt worden. Damit kann eine Apherese-Behandlung wiederholt an ein und demselben Tier vorgenommen werden. Die eingesetzten Tiere können daruberhinaus auch noch in Nachfolgestudien für die Langzeit-Evaluierung der Apherese-Therapie aufgenommen werden. Die Anwendung dieses experimentellen Apherese-Systems wurde erfolgreich an verschiedenen Ratten-Stämmen demonstriert. Wiederholte Apherese-Behandlung wurde in Ratten mit Typ-1-Diabetes und Kollagen Typ II-induzierter Arthritis gut vertragen, wenn ihr Körpergewicht über 250 g lag.

Vor Beginn der experimentellen Apherese-Therapie werden die Tiere mit arteriellen und venösen Kathedern versehen. In einem ersten Schritt werden bei der Apherese zunächst Blutzellen und Plasma mittels Plasmafilter getrennt. Während die Blutzellen unmittelbar in das Tier refundiert werden (über den venösen Katheder) wird das getrennte Plasma an dem in Beispiel 1 hergestellten Adsorptions-Mittel vorbeigeführt (wobei die Liganden durch Bindung an die immobilisierten Rezeptoren aus dem Plasma abgetrennt werden), bevor es dem Tier wieder zugeführt wird.

Alternativ kann auch eine Vollblut-Apherese durchgeführt werden, beispielsweise analog dazu, wie es bei der DALI-Apherese für LDL gemacht wird.

### 3. Erfindungsgemäße Kombinationstherapie im Tiermodell:

Die Kombinationstherapie im Tiermodell kann grundsätzlich so erfolgen, dass der Aß-Efflux vor, während oder nach der Apherese erfolgt. Desweiteren kann die Häufigkeit der Anwendungen der beiden Therapien zueinander variiert werden.

## Patentansprüche

1. Set zur Verwendung zur Vorbeugung oder Behandlung der Alzheimer'schen Erkrankung (AE), umfassend
- ein Mittel zur Induzierung einer Sequestration von Amyloid β (Aβ) in Plasma, wobei das Mittel zur Induzierung einer Sequestration von Aβ in Plasma ausgewählt ist aus Gelsolin, GM1, Aβ-spezifischen Antiköpern, oder einer Aß-spezifischen aktiven oder passiven Vakzine und
- eine Apherese-Vorrichtung, umfassend einen mit dem Blut-oder Plasmafluss kontaktierbaren festen Träger, der einen Amyloid-β-Precurser-Protein (APP) bindenden Rezeptor aufweist, wobei der APP bindende Rezeptor ausgewählt ist aus anti-APP Antikörpern, anti Aβ Antikörpern, insbesondere anti-Aß40-Antikörpern oder anti-Aβ42-Antikörpern, APP bindenden Proteinen Gelsolin, apoJ oder apoE, APP bindenden Gangliosiden, insbesondere GM1, APP bindenden Aptameren, oder Mischungen dieser Rezeptoren,
wobei das Mittel zur Induzierung einer Sequestration von Amyloid β (Aβ) in Plasma an eine Person verabreicht wird und die Person mit der Apherese-Vorrichtung, umfassend einen mit dem Blut- oder Plasmafluss kontaktierbaren festen Träger, der einen Amyloid-β-Precurser-Protein (APP) bindenden Rezeptor aufweist, im extra-korporalen Kreislauf behandelt wird, wobei aus dem Blut der Person APP mittels der Apherese-Vorrichtung entfernt wird.

2. Set zur Verwendung nach Anspruch 1, **dadurch gekennzeichnet, dass** der Träger eine sterile und pyrogenfreie Säule ist.

3. Set zur Verwendung nach Anspruch 1 oder 2, **dadurch gekennzeichnet, dass** die Aß-spezifische Vakzine aus einem Peptid, ausgewählt aus DKELR, DAEFRWP, SAEFRAT, SYEFRHH, SLEFRF, GPEFRW, EIDYHR, ELDYHR, EVDYHR, DIDYHR, DLDYHR, DVDYHR, DIDYRR, DLDYRR, DVDYRR, DKELRI, DWELRI, YREFFI, YREFRI, YAEFRG, EAEFRG, DYEFRG, ELEFRG, DRELRI, DKELKI, DRELKI, GREFRN, EYEFRG, DWEFRDA, SWEFRT, DNEFRSP, SAEFRTQ, SWEFRNP, SWEFRLY, SWELRQA, SVEFRYH, SQEFRTP, SSEFRVS, DWEFRD, DAELRY, DWELRQ, GKEFRT oder SFEFRG, besteht oder ein Peptid, ausgewählt aus DKELR, DAEFRWP, SAEFRAT, SYEFRHH, SLEFRF, GPEFRW, EIDYHR, ELDYHR, EVDYHR, DIDYHR, DLDYHR, DVDYHR, DIDYRR, DLDYRR, DVDYRR, DKELRI, DWELRI, YREFFI, YREFRI, YAEFRG, EAEFRG, DYEFRG, ELEFRG, DRELRI, DKELKI, DRELKI, GREFRN, EYEFRG, DWEFRDA, SWEFRT, DNEFRSP, SAEFRTQ, SWEFRNP, SWEFRLY, SWELRQA, SVEFRYH, SQEFRTP, SSEFRVS, DWEFRD, DAELRY, DWELRQ, GKEFRT oder SFEFRG, umfasst.

## Claims

1. A kit for use in preventing or treating Alzheimer's Disease (AD), comprising
a means for inducing a sequestration of amyloid β (Aβ) in plasma, wherein said means for inducing a sequestration of Aβ in plasma is selected from gelsolin, GM1, Aβ-specific antibodies or an Aβ-specific active or passive vaccine, and
an apheresis device that comprises a solid carrier which can be brought into contact with the blood or with the plasma flux, which carrier has a receptor that binds the amyloid β precursor protein (APP), wherein said receptor that binds APP is selected from anti-APP antibodies, anti-Aβ antibodies, in particular anti-Aß40 antibodies or anti-Aß42 antibodies, the APP binding proteins gelsolin, apoJ or apoE, APP binding gangliosides, in particular GM1, APP binding aptamers, or mixtures of said receptors,
wherein said means for inducing a sequestration of amyloid β (Aβ) in plasma is administered to a person and said person is treated with the apheresis device, which comprises a solid carrier which can be brought into contact with the blood or with the plasma flux that has a receptor that binds the amyloid β precursor protein (APP), in the extracorporeal circulation, wherein APP is removed from the person's blood by means of the apheresis device.

2. Kit for use according to claim 1, **characterized in that** the carrier is a sterile and pyrogen-free column.

3. Kit for use according to claim 1 or 2, **characterized in that** the Aβ-specific vaccine consists of a peptide selected from DKELR, DAEFRWP, SAEFRAT, SYEFRHH, SLEFRF, GPEFRW, EIDYHR, ELDYHR, EVDYHR, DIDYHR, DLDYHR, DVDYHR, DIDYRR, DLDYRR, DVDYRR, DKELRI, DWELRI, YREFFI, YREFRI, YAEFRG, EAEFRG, DYEFRG, ELEFRG, DRELRI, DKELKI, DRELKI, GREFRN, EYEFRG, DWEFRDA, SWEFRT, DNEFRSP, SAEFRTQ, SWEFRNP, SWEFRLY, SWELRQA, SVEFRYH, SQEFRTP, SSEFRVS, DWEFRD, DAELRY, DWELRQ, GKEFRT or SFEFRG or comprises a peptide selected from DKELR, DAEFRWP, SAEFRAT, SYEFRHH, SLEFRF, GPEFRW, EIDYHR, ELDYHR, EVDYHR, DIDYHR, DLDYHR, DVDYHR, DIDYRR, DLDYRR, DVDYRR, DKELRI, DWELRI, YREFFI, YREFRI, YAEFRG, EAEFRG, DYEFRG, ELEFRG, DRELRI, DKELKI, DRELKI, GREFRN, EYEFRG, DWEFRDA, SWEFRT, DNEFRSP, SAEFRTQ, SWEFRNP, SWEFRLY, SWELRQA, SVEFRYH, SQEFRTP, SSEFRVS, DWEFRD, DAELRY, DWELRQ, GKEFRT or SFEFRG.

## Revendications

1. Ensemble destiné à être utilisé pour la prévention ou le traitement de la maladie d'Alzheimer (AE), comprenant
- un agent pour induire une séquestration d'amyloïde ß (Aß) dans le plasma, où l'agent pour induire une séquestration de Aß dans le plasma est choisi parmi Gelsoline, GM1, les anticorps spécifiques de Aß ou un vaccin actif ou passif spécifique de Aß et
- un dispositif d'aphérèse comprenant un support solide pouvant être mis en contact avec la circulation de sang ou de plasma, qui présente un récepteur liant la protéine précurseur d'amyloïde β (APP), où le récepteur liant APP est choisi parmi les anticorps anti-APP, les anticorps anti-Aβ, en particulier les anticorps anti-Aβ40 ou les anticorps anti-Aβ42, les protéines liant APP Gelsoline, apoJ ou apoE, les gangliosides liant APP, en particulier GM1, les aptamères liant APP, ou les mélanges de ces récepteurs,
où l'agent pour induire une séquestration d'amyloïde ß (Aß) dans le plasma est administré à une personne et la personne est traitée avec le dispositif d'aphérèse, comprenant un support solide pouvant être mis en contact avec la circulation de sang ou de plasma, qui présente un récepteur liant la protéine précurseur d'amyloïde β (APP), dans le circuit extracorporel, où APP est retirée du sang de la personne au moyen du dispositif d'aphérèse.

2. Ensemble destiné à être utilisé selon la revendication 1, **caractérisé en ce que** le support est une colonne stérile et sans pyrogènes.

3. Ensemble destiné à être utilisé selon la revendication 1 ou 2, **caractérisé en ce que** le vaccin spécifique de Aß consiste en un peptide choisi parmi DKELR, DAEFRWP, SAEFRAT, SYEFRHH, SLEFRF, GPEFRW, EIDYHR, ELDYHR, EVDYHR, DIDYHR, DLDYHR, DVDYHR, DIDYRR, DLDYRR, DVDYRR, DKELRI, DWELRI, YREFFI, YREFRI, YAEFRG, EAEFRG, DYEFRG, ELEFRG, DRELRI, DKELKI, DRELKI, GREFRN, EYEFRG, DWEFRDA, SWEFRT, DNEFRSP, SAEFRTQ, SWEFRNP, SWEFRLY, SWELRQA, SVEFRYH, SQEFRTP, SSEFRVS, DWEFRD, DAELRY, DWELRQ, GKEFRT ou SFEFRG, ou comprend un peptide choisi parmi DKELR, DAEFRWP, SAEFRAT, SYEFRHH, SLEFRF, GPEFRW, EIDYHR, ELDYHR, EVDYHR, DIDYHR, DLDYHR, DVDYHR, DIDYRR, DLDYRR, DVDYRR, DKELRI, DWELRI, YREFFI, YREFRI, YAEFRG, EAEFRG, DYEFRG, ELEFRG, DRELRI, DKELKI, DRELKI, GREFRN, EYEFRG, DWEFRDA, SWEFRT, DNEFRSP, SAEFRTQ, SWEFRNP, SWEFRLY, SWELRQA, SVEFRYH, SQEFRTP, SSEFRVS, DWEFRD, DAELRY, DWELRQ, GKE-FRT ou SFEFRG.
